# EUROPÄISCHE PATENTANMELDUNG

(11) **EP 1 894 546 A1**
(43) Veröffentlichungstag der Anmeldung: **05.03.2008**
(21) Anmeldenummer: 07015473.7
(22) Anmeldetag: 07.08.2007
(51) Int. Cl.: A61F 5/01

(54) **Handgelenk-Orthese**

(30) Priorität: 29.08.2006 DE 202006013694 U
(71) Anmelder: Bort GmbH, 71384 Weinstadt (DE)
(72) Erfinder: Bort, Wolfgang, 71384 Weinstadt (DE)
(74) Vertreter: Wilhelm, Martin

(57) **Zusammenfassung**

Handgelenk-Orthese mit einer wenigstens einen Armbereich (90) und einen Handbereich (92) aufweisenden Schiene (12), wobei der Armbereich (90) der Schiene (12) wenigstens am rumpffernen Bereich des Unterarms an der Seite des Unterarms anliegt, die in der anatomischen Normalstellung des Armes der Rumpfmittelebene zugewandt ist, und der Handbereich (92) der Schiene (12) wenigstens teilweise an der Handinnenfläche anliegt, wobei an der Seite (96) der Schiene (12), die bezüglich des Unterarms auf der Seite der Elle liegt, sowie an der Seite (94) der Schiene (12), die bezüglich des Unterarms auf der Seite der Speiche liegt, wenigstens drei flügelartige Abschnitte (80, 82, 84, 86, 88) angeordnet sind, von denen wenigstens zwei (80, 82, 84, 86) am Armbereich (90) so zum Umgreifen des Unterarms angeordnet sind, dass der Unterarm wenigstens abschnittsweise sowohl ellenseitig als auch speichenseitig von den flügelartigen Abschnitten (80, 82, 84, 86) umgriffen ist.

Erfindungsgemäß umgreift wenigstens ein flügelartiger Abschnitt (88) am Handbereich (92) die Hand ellenseitig zumindest teilweise.

## Beschreibung

Die Erfindung betrifft eine Handgelenk-Orthese mit einer wenigstens einen Armbereich und einen Handbereich aufweisenden Schiene, wobei der Armbereich der Schiene wenigstens am rumpffernen Bereich des Unterarms an der Seite des Unterarms anliegt, die in der anatomischen Normalstellung des Armes der Rumpfmittelebene zugewandt ist, und der Handbereich der Schiene wenigstens teilweise an der Handinnenfläche anliegt, wobei an der Seite der Schiene, die bezüglich des Unterarms auf der Seite der Elle liegt, sowie der Seite der Schiene, die bezüglich des Unterarms auf der Seite der Speiche liegt, wenigstens vier flügelartige Abschnitte angeordnet sind, von denen wenigstens drei am Armbereich so zum Umgreifen des Unterarms angeordnet sind, dass der Unterarm wenigstens abschnittsweise sowohl ellenseitig als auch speichenseitig von den flügelartigen Abschnitten umgriffen ist.

Im Rahmen dieser Offenbarung werden Begriffe verwendet, deren Bedeutung im Folgenden erläutert und festgelegt wird, wobei sich die Bezeichnungen auf die menschliche Anatomie und Physiologie beziehen und Lage- sowie Richtungsbezeichnungen beziehungsweise Bezeichnungen von Knochen am und im menschlichen Körper darstellen. Die Verwendung von Lage- und Richtungsbezeichnungen beziehungsweise die Benennung von menschlichen Knochen schränkt jedoch die Anwendung der erfindungsgemäßen Handgelenk-Orthese nicht auf den Menschen ein.

Als Radius wird die Speiche im Unterarm bezeichnet. Entsprechend ist unter radial zu verstehen, dass etwas dem Radius zugewandt ist beziehungsweise auf der Seite des Armes einschließlich der Hand angeordnet ist, die dem Radius zugewandt ist. In Normalstellung des Armes verläuft im Unterarm die Ulna, die Elle, im Wesentlichen parallel zum Radius. Unter ulnar ist dementsprechend zu verstehen, dass etwas der Ulna zugewandt ist beziehungsweise auf der Seite des Armes einschließlich der Hand angeordnet ist, die der Ulna zugewandt ist.

Unter der Richtungsangabe proximal wird verstanden, dass etwas dem Körperrumpf zugewandt ist beziehungsweise ein Abschnitt eines Körperteils ist, der nah zur Körpermitte hin gelegen ist. Das Gegenteil von proximal ist distal. So ist ein distaler Bereich vom Körperrumpf abgewandt beziehungsweise ist ein distaler Abschnitt eines Körperteils ein Abschnitt, der weiter von der Körpermitte weg gelegen ist. Eine weitere Richtungsbezeichnung ist dorsal, worunter verstanden wird, dass etwas zur Rückseite eines Körperteils hin orientiert ist, beispielsweise zur Rückseite der Hand oder des Unterarms. Das Gegenteil von dorsal ist ventral, nämlich zur Vorderseite eines Körperteils hin orientiert. Unter volar ist eine Richtungsangabe im Bereich der Hand zu verstehen, gemäß der etwas an der Hand oder am Arm hohlhandseitig angeordnet ist. Volar ist ein Synonym von palmar.

Unter Flexion wird die aktive oder passive Beugebewegung eines Gelenks verstanden. Die Gegenbewegung zur Flexion ist die Extension, also die aktive oder passive Streckbewegung eines Gelenkes. Eine Pronationsbewegung der Hand ist die Drehbewegung des Unterarms, durch die die Daumenseite der Hand zur Körpermittelebene hin, also nach medial, und der Handrücken nach vorne gedreht wird. Die Gegenbewegung zur Pronation ist eine Supinationsbewegung, nämlich eine Drehbewegung des Unterarms, durch die die Daumenseite von der Körpermittelebene weg, also nach lateral, und der Handrücken nach hinten gedreht wird. Eine weitere Bewegungsbezeichnung ist die Abduktion. Darunter wird das seitliche Wegführen oder Abspreizen eines Körperteils von der Körpermitte oder der Längsachse einer Extremität in der Frontalansicht des Körpers beziehungsweise der Extremität verstanden. Die Gegenbewegung zur Abduktion ist die Adduktion.

Gattungsgemäße Handgelenk-Orthesen sind beispielsweise aus der DE 94 01 355 U1 bekannt. Die darin beschriebene Orthese besteht aus einer Schiene, die einen Armbereich und einen Handbereich aufweist. Der Armbereich liegt wenigstens am distalen Bereich des Unterarms volar an. Der Handbereich liegt wenigstens teilweise an der Handinnenfläche an. An der ulnaren Seite der Schiene sowie an der radialen Seite der Schiene sind am Armbereich drei flügelartige Abschnitte angeordnet. Die flügelartigen Abschnitte sind so zum Umgreifen des Unterarms angeordnet, dass der Unterarm wenigstens abschnittsweise sowohl ulnar als auch radial, also bilateral von den flügelartigen Abschnitten umgriffen ist. Derartige Handgelenk-Orthesen werden für die Ruhigstellung von Handgelenken verwendet, insbesondere für die Unterbindung von Flexionsbewegungen und Extensionsbewegungen. Alle anderen Bewegungen, die mit dem Handgelenk durchgeführt werden können, sind noch möglich, insbesondere die Ulnarabduktion sowie die Radialabduktion.

Die der Erfindung zugrundeliegende Aufgabe ist es, eine Handgelenk-Orthese zu schaffen, mit der eine Ulnarabduktion unterbunden sowie eine ulnare Deviation, also eine ulnar ausgerichtete Fehlstellung der Hand, behandelt werden kann, die in der Anwendung einfach ist und einen hohen Tragekomfort aufweist.

Gelöst wird diese Aufgabe durch eine Handgelenk-Orthese mit den Merkmalen von Anspruch 1. Vorteilhafte sowie bevorzugte Ausgestaltungen der Erfindung sind Gegenstand der weiteren Ansprüche und werden im Folgenden näher erläutert. Manche der nachfolgenden, jedoch nicht erschöpfend aufgezählten Merkmale und Eigenschaften werden teilweise nur einmal beschrieben, können jedoch, ohne den Rahmen der Erfindung zu verlassen, beliebig kombiniert werden. Weiterhin ist die Reihenfolge der aufgelisteten Merkmale nicht bindend, sondern kann vielmehr entsprechend einer optimierten Handgelenk-Orthese geändert werden.

Erfindungsgemäß ist eine Handgelenk-Orthese mit einer wenigstens einen Armbereich und einen Handbereich aufweisenden Schiene vorgesehen, wobei der Armbereich der Schiene wenigstens am rumpffernen Bereich des Unterarms an der Seite des Unterarms anliegt, die in der anatomischen Normalstellung des Armes der Rumpfmittelebene zugewandt ist, und der Handbereich der Schiene wenigstens teilweise an der Handinnenfläche anliegt, wobei an der Seite der Schiene, die bezüglich des Unterarms auf der Seite der Elle liegt, sowie der Seite der Schiene, die bezüglich des Unterarms auf der Seite der Speiche liegt, wenigstens drei flügelartige Abschnitte angeordnet sind, von denen wenigstens zwei am Armbereich so zum Umgreifen des Unterarms angeordnet sind, dass der Unterarm wenigstens abschnittsweise sowohl ellenseitig als auch speichenseitig von den flügelartigen Abschnitten umgriffen ist, wobei wenigstens ein flügelartiger Abschnitt am Handbereich die Hand ellenseitig zumindest teilweise umgreift.

Die Anwendung einer erfindungsgemäßen Handgelenk-Orthese kann beispielsweise bei einem so genannten Karpaltunnelsyndrom (CTS), bei Discusverletzungen im Ulno-Karpal-Gelenk, bei Arthrosen der Handwurzel, Bänderläsionen im Handwurzelbereich sowie bei Distorsion, Arthralgien, Rheuma und bei der so genannten Fallhand indiziert sein. Die erfindungsgemäße Handgelenk-Orthese kann darüber hinaus beispielsweise zur postoperativen Ruhigstellung und/oder zur funktionellen Beübung in einem Zustand nach Mittelhandfrakturen Anwendung finden. Es ist weiterhin möglich mit der erfindungsgemäßen Handgelenk-Orthese eine ulnare Deviation des Handgelenks zu behandeln.

Besonders vorteilhaft zeigt sich, dass die erfindungsgemäße Handgelenk-Orthese nicht nur für die Therapierung einer Vielzahl von Krankheitsbildern herangezogen werden kann, sondern auch einen hohen Tragekomfort aufweist. Dies insbesondere deshalb, da die Schiene nur weniges Anlagepunke am Arm beziehungsweise der Hand aufweist und somit nur wenig Haut von der Schiene bedeckt ist. Auch das Anlegen der Handgelenk-Orthese ist sehr vereinfacht, da die Hand lediglich ausgehend von der proximalen Seite der Handgelenk-Orthese handschuhartig in die Handgelenk-Orthese eingeschoben werden muss, bis die Handgelenk-Orthese an ihrer angedachten Position liegt.

Die verwendete Schiene ist dazu vorzugsweise im Wesentlichen steif und länglich ausgebildet, so dass die Schiene im Zusammenwirken mit den flügelartigen Abschnitten eine Bewegung im Handgelenk unterbinden kann. Die Schiene erstreckt sich dazu vorzugsweise ausgehend vom Handgelenk in Richtung des proximalen Unterarms und bildet so den Armbereich. Der Armbereich ist so lang zu wählen, dass die möglichen Bewegungen im Handgelenk unterbunden beziehungsweise die dazu aufgebrachten Kräfte mittels der flügelartigen Abschnitte in den Unterarm eingebracht werden können, ohne dass die in den Unterarm eingebrachten Kräfte als unangenehm empfunden werden oder Druckstellen erzeugen können.

Die flügelartigen Abschnitte können beispielsweise im Wesentlichen länglich ausgebildet sein, so dass sie eine möglichst große Auflagefläche am Unterarm beziehungsweise an der Hand aufweisen. Sie können verschiedenen Formen aufweisen, beispielsweise im wesentlichen rechteckig oder halbkreisförmig, wobei jede Form denkbar ist, die im Rahmen der Erfindung als sinnvoll erscheint. Das Umgreifen des Unterarms im Armbereich beziehungsweise der Hand an der ulnaren Handseite im Handbereich durch die flügelartigen Abschnitte bedeutet, dass sich die flügelartigen Abschnitte von der volaren Hand- beziehungsweise Armseite in Richtung der dorsalen Hand- beziehungsweise Armseite erstrecken und dadurch im wesentlichen einen Klammereffekt erzielen.

Durch das Vorsehen wenigstens eines flügelartigen Abschnitts im Handbereich an der ulnaren Seite der Hand und wenigstens zweier flügelartiger Abschnitte im Armbereich wird im Zusammenspiel der flügelartigen Abschnitte mit der Schiene eine ausreichende Stabilisierung des Handgelenks erreicht, wenn die flügelartigen Abschnitte wechselseitig am Wirkungsbereich der Handgelenk-Orthese vorgesehen sind. Im Verlauf der Schiene, ausgehend vom Handbereich in Richtung Körperrumpf sind die flügelartigen Abschnitte also vorzugsweise ulnar, radial und wieder ulnar angeordnet.

Die Schiene kann am Unterarm und an der Hand beispielsweise mittels eines elastischen Verbandes fixiert werden.

In Weiterbildung der Erfindung weist die Schiene vier flügelartige Abschnitte am Armbereich und einen flügelartigen Abschnitt am Handbereich auf.

Durch das Vorsehen von vier flügelartigen Abschnitten im Armbereich wird der Tragekomfort sowie die Stabilisierung des Handgelenks in Zusammenwirkung der vier flügelartigen Abschnitte mit dem einen flügelartigen Abschnitt im Handbereich weiter erhöht. Die vier flügelartigen Abschnitte sind dazu vorzugsweise paarweise bilateral am Armbereich angeordnet. So kann beispielsweise ein erstes Paar der flügelartigen Abschnitte im Bereich des Handgelenks das Handgelenk bilateral, also sowohl radial als auch ulnar umgreifen. Ein zweites Paar der flügelartigen Abschnitte kann im proximalen Bereich des Unterarms vorgesehen sein und den Unterarm in gleicher Weise umgreifen, wie die flügelartigen Abschnitte im Bereich des Handgelenks das Handgelenk.

In Weiterbildung der Erfindung weist die Schiene einen länglich ausgebildeten Mittelsteg auf, der sich über den Armbereich und den Handbereich der Schiene erstreckt und mit dem die flügelartigen Abschnitte verbunden sind, insbesondere einstückig mit dem Mittelsteg an den Längsseiten des Mittelsteges.

Die flügelartigen Abschnitte können dazu mit dem Mittelsteg verschweißt, verklebt, vernietet oder auf eine andere Weise verbunden sein. Es ist jedoch auch denkbar, den Mittelsteg und die flügelartigen Abschnitte als ein einziges Stanzteil oder dergleichen herzustellen, so dass keine Kanten zwischen den flügelartigen Abschnitten und dem Mittelsteg entstehen, beziehungsweise keine weiteren Arbeitsschritte für den Zusammenbau der flügelartigen Abschnitte mit dem Mittelsteg nötig sind.

In Weiterbildung der Erfindung besteht die Schiene aus einem Metall, insbesondere aus Aluminium, und/oder aus einem Kunststoff.

Durch das Vorsehen von Aluminium ist die Schiene leicht und darüber hinaus korrosionsbeständig. Das Stanzen beziehungsweise Pressen von Aluminium ist ein gut beherrschbarer Vorgang, so dass materialbedingte Voraussetzungen für ein Medizinprodukt erfüllt sind. Die Verwendung von Kunststoffen für die Schiene weist ähnliche Vorteile auf, nämlich das geringe Gewicht bei gleichzeitig ausreichender Stabilität der Schiene.

In Weiterbildung der Erfindung erstreckt sich der flügelartige Abschnitt am Handbereich ausgehend vom Bereich der Handinnenfläche über den Bereich der Handkante und zumindest teilweise bis in den Bereich des Handrückens.

Dadurch wird erreicht, dass keine Kante des flügelartigen Abschnitts in einem Bewegungsbereich beziehungsweise in einer Bewegungsrichtung der Hand liegt, wodurch Druckstellen vermieden und der Tragekomfort der Handgelenk-Orthese verbessert wird. Darüber hinaus ist durch diesen Verlauf des flügelartigen Abschnitts im Handbereich erreicht, dass eine Ulnarabduktion der Hand unterbunden wird beziehungsweise einer ulnaren Deviation der Hand entgegengewirkt wird.

In Weiterbildung der Erfindung sind die flügelartigen Abschnitte zum Anpassen an die Kontur des Arms beziehungsweise die Kontur der Hand biegbar.

Auf diese Weise können die flügelartigen Abschnitte an anatomisch unterschiedliche Konturen der Unterarme und Hände von verschiedenen Menschen angepasst werden. Es kann also die eine Handgelenk-Orthese einer Größe an unterschiedliche anatomische Gegebenheiten angepasst werden.

In Weiterbildung der Erfindung sind die flügelartigen Abschnitte rahmenartig ausgebildet und weisen eine von dem Rahmen begrenzte Ausnehmung auf.

Durch die Ausnehmung wird eine weitere Gewichtsreduzierung der Handgelenk-Orthese erreicht, insbesondere der Schiene mit den flügelartigen Abschnitten, so dass der Tragekomfort wiederum weiter erhöht wird. Auch ist die Biegesteifigkeit der flügelartigen Abschnitte durch das Vorsehen der Ausnehmungen verringert, so dass das Anpassen der flügelartigen Abschnitte an die anatomischen Gegebenheiten keiner speziellen Werkzeuge bedarf, sonder manuell und werkzeuglos durchgeführt werden kann.

In Weiterbildung der Erfindung verläuft der Mittelsteg der Schiene im Wesentlichen parallel zum Unterarm. Die Schiene ist an den Verlauf der Kontur von Unterarm, Handgelenk und Mittelhand anpassbar und/oder angepasst, insbesondere in deren anatomischer Normalstellung, in der von der Achse des Radius und der Achse der Mittelhandknochen etwa ein Winkel von 35° gebildet wird. Der Handbereich ist an die Form der Handinnenfläche angepasst, insbesondere tellerartig, O-förmig, L-förmig, T-förmig oder V-förmig.

In Weiterbildung der Erfindung ist die Schiene wenigstens teilweise mit Leder und/oder einem Textil oder dergleichen überzogen, wobei der Überzug zumindest abschnittsweise atmungsaktiv und/oder feuchtigkeitsableitend ist und wobei der Überzug zumindest teilweise mit einer Polsterung versehen ist, insbesondere im Bereich der Schiene und/oder den flügelartigen Abschnitten.

Der Überzug ist vorzugsweise so ausgebildet, dass er den Unterarm und die Hand, abgesehen von einem vorzugsweise durchgehenden dorsalen Spalt entlang der Längsausdehnung der Handgelenk-Orthese, im Wesentlichen vollständig einhüllen kann. Die Einhüllung von Unterarm und Hand erhöht die Stabilisierung des Handgelenks zusätzlich zur stabilisierenden Wirkung der Schiene und der flügelartigen Abschnitte. Das Vorsehen von Polsterungen im und/oder am Überzug erhöht wiederum den Tragekomfort weiter und unterbindet insbesondere im Bereich der Schiene und der flügelartigen Abschnitte Druckstellen am Unterarm beziehungsweise der Hand. Die Polsterungen beziehungsweise der Überzug können im wesentlichen aus einem beliebigen Material gefertigt sein, das für ein medizintechnisches Produkt der vorliegenden Art zugelassen ist.

In Weiterbildung der Erfindung sind an der Handgelenk-Orthese Verschlussmittel zum Fixieren der Orthese vorgesehen, die wenigstens im Bereich des Unterarms und/oder im Bereich der Hand im Wesentlichen an der dorsalen Seite an der Handgelenk-Orthese angeordnet ist, wobei mittels der Verschlussmittel eine Fixierung im wesentlichen über die gesamte Länge der Handgelenk-Orthese erreicht werden kann.

Durch die Fixierung kann die Handgelenk-Orthese nach Art eines Gipsverbandes an den zu behandelnden Bereich angelegt werden und den Gipsverband einschließlich dessen Vorteile vollständig ersetzen, ohne jedoch die Nachteile eines Gipsverbandes aufzuweisen. Die Fixierung ist vorzugsweise so ausgelegt, dass die Schiene eventuell einschließlich des Überzugs dicht an den zu behandelnden Bereich angelegt und ein Verrutschen der Handgelenk-Orthese vermieden werden kann. Die Effektivität der erfindungsgemäßen Handgelenk-Orthese steigt, wenn sie die entsprechenden Bewegungen zuverlässig unterbinden kann, was durch die vorgesehene Fixierung unterstützt wird.

In Weiterbildung der Erfindung weisen die Verschlussmittel wenigstens ein Band, wenigstens einen Riemen und/oder einen Haken auf, wobei das wenigstens eine Band, der wenigstens eine Riemen und/oder der Haken mit wenigstens einer Schlaufe in Eingriff steht, die an den flügelartigen Abschnitten, dem Überzug und/oder der Schiene angeordnet ist und dass das wenigstens eine Band und der wenigstens eine Riemen mit einem Klettband beziehungsweise einer Schnalle versehen ist.

In einer Ausgestaltung der Erfindung weist die Handgelenk-Orthese mehrere Bänder und/oder Riemen auf, die beabstandet zueinander im Wesentlichen senkrecht zum Unterarm im Armbereich der Schiene angeordnet sind, wobei wenigstens ein erstes Band oder Riemen an der der Elle zugewandten Seite der Schiene fixiert ist und wenigstens ein zweites Band oder Riemen an der der Speiche zugewandten Seite der Schiene fixiert ist.

Das wenigstens eine für die Fixierung vorgesehene Band, wobei Band im Folgenden gleichbedeutend mit Riemen verwendet wird, ist beispielsweise senkrecht zum Verlauf der Handgelenk-Orthese über den gesamten Verlauf des Überzugs mit dem Überzug der Handgelenk-Orthese verbunden, insbesondere vernäht. Dabei ist ein Endbereich des Bandes mit einer Schnalle versehen, die im Bereich des vorstehend beschriebenen Spaltes angeordnet ist. Das Band weist insgesamt etwa die doppelte Länge auf, die für das Vernähen mit dem Überzug nötig ist, so dass der andere, freie Endbereich des Bandes deutlich über den Spalt übersteht. Für die Fixierung wird nun der freie Endbereich den Spalt übergreifend durch die entsprechenden Schlaufe am nicht freien Endbereich des Bandes geführt und durch die Schlaufe umgelenkt, so dass das Band wieder den Spalt übergreifen kann. Mittels des Bandes, der Schlaufe und der Vernähung des Bandes kann der Spalt zugezogen werden und somit die Handgelenk-Orthese fest den Unterarm umschließend angelegt werden.

In Weiterbildung der Erfindung umgibt im Armbereich wenigstens eines der Bänder im verschlossenen Zustand den Arm im Bereich des Handgelenks vollständig und ist so breit, dass es den Bereich des Handgelenks in Längsrichtung des Arms gesehen im wesentlichen vollständig überdeckt.

Die im Wesentlichen vollständige Überdeckung des Handgelenks erhöht die stabilisierende Wirkung der Handgelenk-Orthese noch weiter. Dieses breite Band wird vorteilhafterweise vollständig um den Unterarm gewickelt, um eine sichere Fixierung zu gewährleisten.

In Weiterbildung der Erfindung ist eine Daumen-Schlaufe für die Fixierung der Handgelenk-Orthese relativ zum Daumen vorgesehen.

Die Möglichkeit eines Verrutschens der Handgelenk-Orthese, beispielsweise um die Achse des Unterarms oder parallel zu dieser, kann so verringert werden.

In Weiterbildung der Erfindung ist die Daumen-Schlaufe aus einem Streifen des Materials eines Überzugs gebildet, wobei ein erster Endbereich des Streifens in einem distalen Bereich der Handgelenk-Orthese mit dem Überzug unlösbar verbunden ist und der Streifen in seinem zweiten Endbereich mit der Handgelenk-Orthese lösbar verbunden ist, insbesondere mittels eines Klettbandes oder dergleichen.

Das Anlegen der Handgelenk-Orthese wird auf diese Weise weiter vereinfacht, da die Schlaufe während des Anlegens geöffnet sein kann und erst dann, wenn die Handgelenk-Orthese korrekt am Unterarm beziehungsweise der Hand platziert ist, auf einfache Weise mittels des Klettbandes verschlossen wird.

In Weiterbildung der Erfindung sind an dem zweiten Endbereich des Streifens zwei Bänder angeordnet, insbesondere Klettbänder, wobei eines der Bänder den Streifen im Wesentlichen verlängernd angeordnet ist und das zweite Band in einem Winkel zum ersten Band ausgerichtet am Streifen angeordnet ist. Das erste und das zweite Band schließen einen Winkel zwischen 100° und 160° ein, insbesondere 125°.

Das Anordnen dieser beiden Bänder in einem Winkel zueinander ermöglicht ein Verspannen der Schlaufe beziehungsweise der Schlaufe mit dem Überzug in zwei Richtungen, insbesondere nach radial und nach ulnar des Spaltes, so dass der Überzug weiter stabilisiert wird.

In Weiterbildung der Erfindung ist das erste Band im verschlossenen Zustand im Wesentlichen parallel zu einer Linie ausgerichtet, die sich von einem Bereich zwischen den Metakarpal-Phalangelenken von Daumen und Zeigefinger über den Handrücken im Bereich der Mittelhandknochen bis zur Handkante im Bereich des Mittelhandknochens des kleinen Fingers erstreckt. Das zweite Band ist im verschlossenen Zustand im Wesentlichen parallel zu einer Linie ausgerichtet, die vom Metakarpal-Phalangelenk des kleinen Fingers über den Handrücken zum Karpo-Metakarpalgelenk des Daumens verläuft.

Das Metakarpal-Phalangelenk ist das Gelenk der Finger und des Daumens zwischen den distalen Mittelhandknochenbereichen und dem proximalen Bereich der Grundphalanx, also dem ersten Knochen eines Fingers beziehungsweise des Daumens, der distal auf den entsprechenden Mittelhandknochen folgt.

Das Karpo-Metakarpalgelenk ist das Gelenk eines der Mittelhandknochen in dessen proximalem Bereich mit einem der Handwurzelknochen.

Der Verlauf der Bänder im Wesentlichen parallel zu den hier beschriebenen Linien ermöglicht es, eine Radialabduktion der Hand mittels der Daumenschlaufe im Wesentlichen zu unterbinden, so dass insgesamt im Handgelenksbereich alle normalerweise möglichen Bewegungen nur noch in einem sehr geringen Umfang durchführbar sind.

In Weiterbildung der Erfindung sind die beiden Bänder als Klettbänder ausgebildet und an einem textilen Überzug der Schiene befestigbar.

Weitere Vorteile und Merkmale der Erfindung ergeben sich aus den Ansprüchen und der nachfolgenden Beschreibung eines Ausführungsbeispiels der Erfindung, das anhand der Zeichnungen dargestellt ist. Teilweise sind Merkmale nur in Bezug auf eine Abbildung des Ausführungsbeispiels beschrieben, können jedoch im Rahmen der Erfindung beliebig mit den anderen Abbildungen kombiniert werden. In den schematischen Zeichnungen zeigen:
- Fig.1: eine isometrische Ansicht der erfindungsgemäßen Handgelenk-Orthese,
- Fig.2: eine Draufsicht einer Abwicklung einer in der erfindungsgemäßen Handgelenk-Orthese angeordneten Schiene,
- Fig.3: eine isometrische Ansicht der Schiene aus Fig.2, und
- Fig.4: eine dorsale, anatomische Ansicht einer Hand mit abgebildeten Knochen.

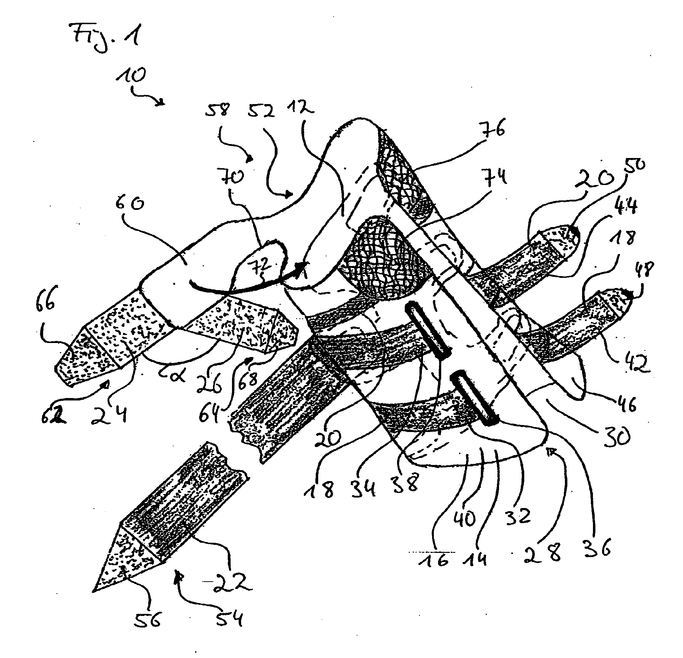

In Fig.1 ist eine isometrische Ansicht einer erfindungsgemäßen Handgelenk-Orthese 10 dargestellt. Die Handgelenk-Orthese 10 weist eine strichliniert dargestellte Schiene 12 sowie einen aus einem Textil bestehenden Überzug 14 auf, der die Schiene 12 in sich aufnimmt. Der Überzug 14 bildet im Wesentlichen eine Umhüllung für einen nicht dargestellten Unterarm sowie eine nicht dargestellte Hand, wobei an der dorsalen Seite 28 der Handgelenk-Orthese durch den Überzug 14 ein Spalt 30 gebildet ist. Auf der Außenseite 16 des Überzugs 14 sind mehrere Bänder 18, 20, 22, 24, 26 mit dem Überzug 14 vernäht, wobei die Bänder 18, 20, 22, 24, 26 zusammen mit Teilen der Handgelenk-Orthese 10, mit denen die Bänder 18, 20, 22, 24, 26 im verschlossenen Zustand in Eingriff stehen, Verschlussmittel der Handgelenk-Orthese 10 bilden.

Das proximale Unterarm-Band 18 und das distale Unterarm-Band 20 sind etwa doppelt so lang wie der Umfang der Handgelenk-Orthese 10 an den Befestigungsbereichen der Bänder 18, 20. Die Bänder 18, 20 sind etwa über die Hälfte ihrer Länge, also im Wesentlichen über den vollen Umfang der Handgelenk-Orthese 10, mit der Handgelenk-Orthese 10 vernäht. In den ersten Endbereichen 32, 34 der Bänder 18, 20 sind an den Bändern 18, 20 Schlaufen 36, 38 befestigt, wobei die ersten Endbereiche 32, 34 im Wesentlichen mit den Endbereichen der Vernähung der Bänder 18, 20 zusammenfallen und auf der radialen Seite 40 der Handgelenk-Orthese 10 liegen. Die zweiten Endbereiche 42, 44 der Bänder 18, 20 weisen etwa die halbe Länge der Bänder 18, 20 auf und liegen auf der ulnaren Seite 46 der Handgelenk-Orthese 10. An die zweiten Endbereiche 42, 44 der Bänder 18, 20 schließen sich Bandabschnitte 48, 50 an, die mit einem ersten Teil eines Klettverschlusses versehen sind und im verschlossenen Zustand der Handgelenk-Orthese 10 mit Abschnitten des jeweiligen Bandes 18, 20 in Eingriff gebracht werden, die selbst klettverschlussartige Eigenschaften aufweisen.

Auf der volaren Seite 52 der Handgelenk-Orthese 10, die der dorsalen Seite 28 gegenüber liegt, ist das Handgelenkband 22 an einer in Fig.1 nicht sichtbaren Stelle mit dem Überzug 14 vernäht. Das Handgelenkband 22 ist in seinem freien Endbereich 54 ebenfalls mit einem Klettbandabschnitt 56 versehen. Die Länge des Handgelenkbandes 22 entspricht wenigstens dem Umfang der Handgelenk-Orthese 10. Das Handgelenkband 22 wird im korrekt angelegten Zustand der Handgelenk-Orthese 10 um die Handgelenk-Orthese 10 geschlungen, wobei es hierzu über die radiale Seite 40 der Handgelenk-Orthese 10, den Spalt 30 und dann über die ulnare Seite 46 der Handgelenk-Orthese 10 geführt wird. Das Klettband 56 des Handgelenkbandes 22, das im Verlauf der korrekt angelegten Handgelenk-Orthese 10 etwa im Bereich des Handgelenks angeordnet ist, wird dann etwa in dem Bereich mit dem vernähten Endbereich des Handgelenkbandes 22 in Eingriff gebracht, in dem es vernäht ist. Das Handgelenkband 22 weist hierzu ebenfalls klettbandartige Eigenschaften auf. Das Handgelenkband 22 ist so breit, dass es in Längsrichtung des Armes gesehen das Handgelenk im Wesentlichen vollständig überdeckt. Da das Handgelenkband 22 auch einmal vollständig um das Handgelenk geschlungen wird, sorgt es für eine zuverlässige Ruhigstellung des Handgelenks und eine sehr stabile Befestigung der Handgelenk-Orthese 10.

Wie in Fig.1 weiter zu erkennen ist, wird beim Schließen der Bänder 18, 20, die durch die Schlaufen 36, 38 gezogen werden, eine Zugkraft von der ulnaren Seite 46 auf die radiale Seite 40 der Handgelenk-Orthese 10 ausgeübt. Beim nachfolgenden Schließen des Handgelenkbandes 22 wird dahingegen eine Zugkraft von der radialen Seite 40 auf die ulnare Seite 46 ausgeübt, also genau umgekehrt. Dadurch lässt sich die Handgelenk-Orthese 10 besonders zuverlässig und fest anlegen.

Am distalen Ende 58 der Handgelenk-Orthese 10 ist am Überzug 14 ein Streifen 60 vorgesehen. An dem Streifen 60 ist den Streifen 60 verlängernd das Daumenband 24 angebracht. An einem proximalen Seitenbereich des Streifens 60 ist in einem Winkel α von etwa 125° das schräge Daumenband 26 an dem Streifen 60 angebracht. An den Endbereichen 62, 64 der Bänder 24, 26 sind Klettbandabschnitte 66, 68 vorgesehen. Im korrekt angelegten Zustand der Handgelenk-Orthese 10 umschlingt der Streifen 60 im wesentlichen einen nicht dargestellten Daumen, wobei der Daumen dann in einer Schlaufe liegt, die mittels einer Ausnehmung 70 sowie einer Umschlingbewegung des Streifens 60 gemäß Pfeil 72 gebildet wird. In dem den Daumen umschlingenden Zustand des Streifens 60 können die Klettbandabschnitte 66, 68 mit entsprechenden Klettstellen 74, 76 auf der radialen Seite 40 beziehungsweise der ulnaren Seite 46 der Handgelenk-Orthese 10 in Eingriff gebracht werden.

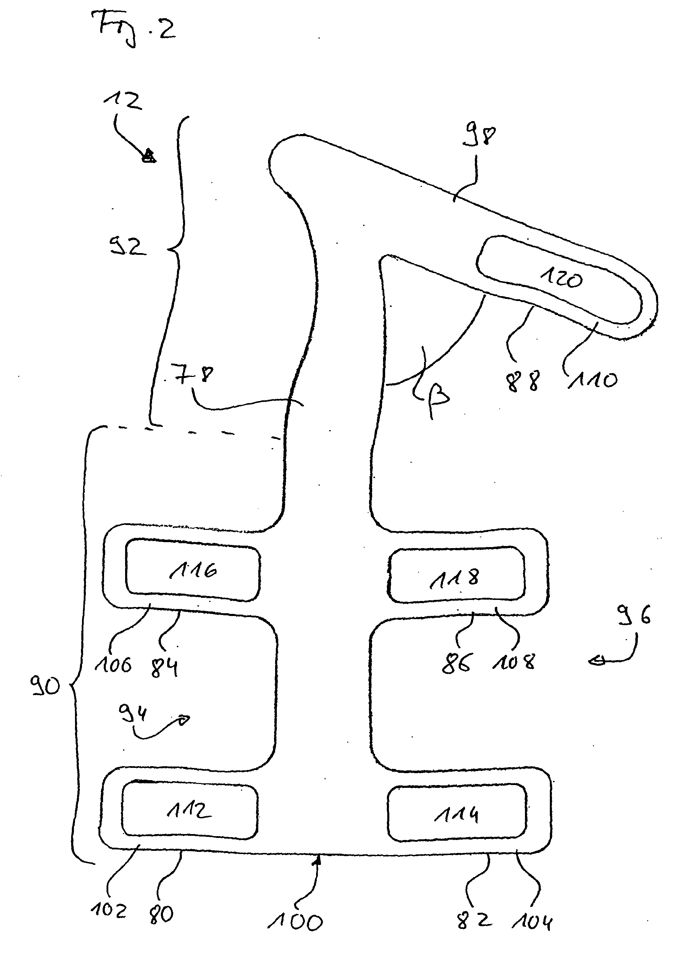

In Fig.2 ist die Schiene 12 der Fig.1 in einer abgewickelten Draufsicht dargestellt. Die Schiene 12 weist einen Mittelsteg 78 auf, von dem flügelartige Abschnitte 80, 82, 84, 86, 88 ausgehen, wobei die flügelartigen Abschnitte im Wesentlichen senkrecht zum Mittelsteg 78 ausgerichtet sind. Die Schiene 12 ist in einen Armbereich 90 und einen Handbereich 92 unterteilt. Im Armbereich 90 sind die flügelartigen Abschnitte 80, 82, 84, 86 jeweils paarweise, nämlich ein erstes Paar 80, 82 und ein zweites Paar 84, 86, an jeweils gegenüberliegenden Seiten der Schiene 12 angeordnet, nämlich an der radialen Seite 94 und der ulnaren Seite 96 der Schiene 12.

Der Handbereich 92 der Schiene 12 ist im Wesentlichen T-förmig ausgebildet, wobei sich der Querbalken 98 der T-Form deutlich weiter nach ulnar, in Fig.2 also rechts, erstreckt als nach radial, in Fig.2 also nach links, und im korrekt angelegten Zustand der Handgelenk-Orthese in etwa medial der Mittelhandknochen positioniert ist. Der Querbalken 98, und somit auch der flügelartige Abschnitt 88, der im Endbereich des Querbalkens 98 angeordnet ist, schließt mit dem Mittelsteg 78 einen Winkel β von etwa 80° ein, wobei sich der Winkel β zum proximalen Ende 100 der Schiene 12 hin öffnet.

Die flügelartigen Abschnitte 80, 82, 84, 86, 88 weisen jeweils einen Rahmen 102, 104, 106, 108, 110 auf, der jeweils eine Ausnehmung 112, 114, 116, 118, 120 umgibt.

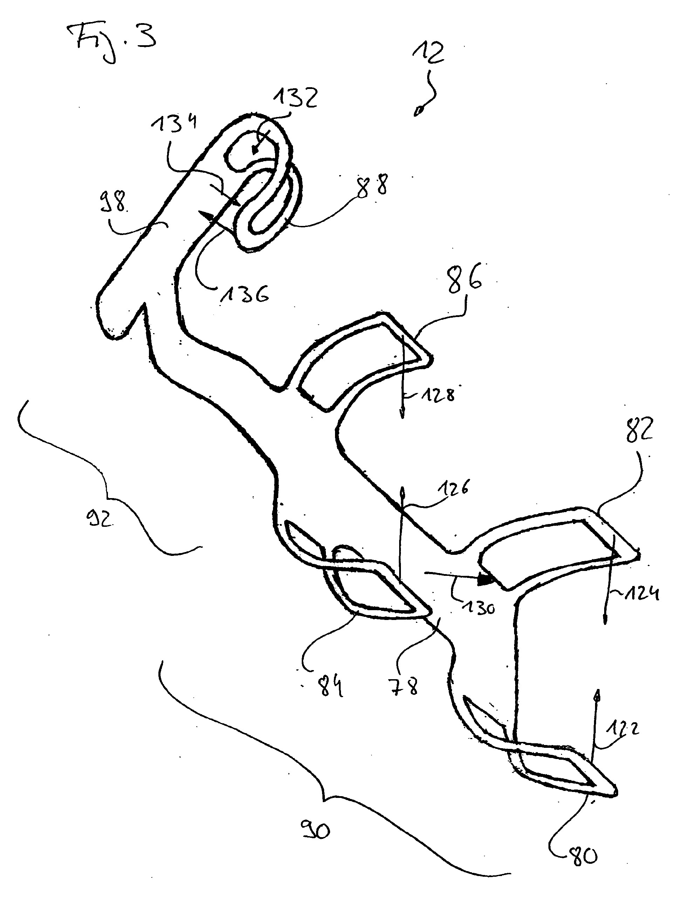

In Fig.3 ist eine isometrische Ansicht der Schiene 12 aus Fig.2 dargestellt, bei der die flügelartigen Abschnitte 80, 82, 84, 86, 88 so gebogen sind, dass sie im korrekt angelegten Zustand der Handgelenk-Orthese einen nicht dargestellten Unterarm ulnar und radial beziehungsweise eine nicht dargestellte Hand ulnar abschnittsweise umgreifen können. Es ist in Fig.3 zu erkennen, dass die flügelartigen Abschnitte 80, 82, 84, 86 im Unterarm-Bereich 90 der Schiene 12 etwa eine Viertelkreisbiegung aufweisen. Dahingegen weist der ulnare flügelartige Abschnitt 88 im Handbereich 92 der Schiene 12 im Wesentlichen eine Halbkreisbiegung auf. Diese unterschiedlichen Biegungen rühren daher, dass im Unterarm-Bereich 90 durch die flügelartigen Abschnitte 80, 82, 84, 86 Kräfte lediglich in Richtung der Pfeile 122, 124, 126, 128, nämlich radial und ulnar, in den Unterarm einleiten müssen. Kräfte, die von volar gemäß Pfeil 130 in den Unterarm eingeleitet werden müssen, werden von dem Bereich des Mittelstegs 78 im Armbereich 92 übertragen.

Im Unterschied zu den flügelartigen Abschnitten 80, 82, 84, 88 ist der flügelartige Abschnitt 88 halbkreisartig gebogen, so dass eine Handkante an der ulnaren Seite der Hand im Wesentlichen vollständig umschlossen ist. Dadurch kann der flügelartige Abschnitt 88 die Hand in drei Richtungen stabilisieren. Das bedeutet, dass der flügelartige Abschnitt 88 Kräfte in Richtung der Pfeile 132, 134, 136 aufnehmen kann, wobei eine Kraftaufnahme aus Richtung des Pfeils 134 noch durch den Querbalken 98 unterstützt wird. Die Aufnahme der Kräfte aus den in Bezug auf Fig.3 noch nicht beschriebenen Richtungen werden mittels dem in Fig.3 nicht dargestellten Überzug aufgenommen.

Der Handbereich 92 ist relativ zum Armbereich 90 etwa in einem Winkel von 35° nach dorsal abgeknickt. Dieser Winkel entspricht im wesentlichen dem Winkel zwischen dem Radius und den Mittelhandknochen der Hand in der normalen Handstellung. Da die Schiene 12 ein Alustanzteil ist, kann dieser Winkel, wie auch die Biegungen der flügelartigen Abschnitte 80, 82, 84, 86, 88, der Anatomie beziehungsweise Physiologie der jeweiligen zu behandelnden Person manuell angepasst werden.

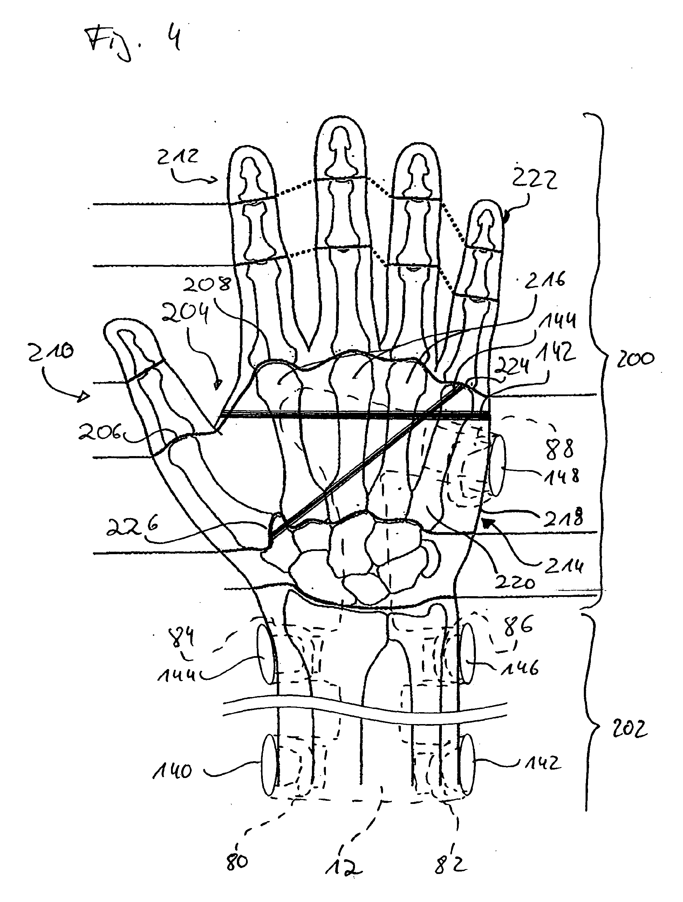

In Fig.4 ist eine dorsale, anatomische Ansicht einer Hand 200 mit sichtbaren knöchernen Strukturen dargestellt. Außerdem ist in Fig.4 dargestellt, wie die strichliniert dargestellte Schiene 12 an der volaren Seite der Hand an der Hand anliegt, beziehungsweise an welchen Anlagebereichen 140, 142, 144, 146, 148 die flügelartigen Abschnitte 80, 82, 84, 86, 88 an der Hand 200 beziehungsweise am Unterarm 202 anliegen.

Im Wesentlichen ist aus Fig.4 jedoch ersichtlich, wie die Daumenbänder 24, 26 der Fig.1 im korrekt angelegten Zustand der Handgelenk-Orthese 10 verlaufen. Das in Fig.4 nicht dargestellte Daumenband 24 der Fig.1 verläuft dann im Wesentlichen parallel zu einer Linie 142 von einem Bereich 204 zwischen den Metakarpal-Phalangelenken 206, 208 von Daumen 210 und Zeigefinger 212 über den Handrücken 214 im Bereich der Mittelhandknochen 216 bis zur Handkante 218 im Bereich des Mittelhandknochens 220 des kleinen Fingers 222. Das in Fig.4 nicht dargestellte schräge Daumenband 26 der Fig.1 verläuft im Wesentlichen parallel zu einer Linie 144, die vom Metakarpal-Phalangelenk 224 des kleinen Fingers 222 über den Handrücken 214 zum Karpo-Metakarpalgelenk 226 verläuft.

## Patentansprüche

1. Handgelenk-Orthese mit einer wenigstens einen Armbereich (90) und einen Handbereich (92) aufweisenden Schiene (12), wobei der Armbereich (90) der Schiene (12) wenigstens am rumpffernen Bereich des Unterarms an der Seite des Unterarms anliegt, die in der anatomischen Normalstellung des Armes der Rumpfmittelebene zugewandt ist, und der Handbereich (92) der Schiene (12) wenigstens teilweise an der Handinnenfläche anliegt, wobei an der Seite (96) der Schiene (12), die bezüglich des Unterarms auf der Seite der Elle liegt, sowie an der Seite (94) der Schiene (12), die bezüglich des Unterarms auf der Seite der Speiche liegt, wenigstens drei flügelartige Abschnitte (80, 82, 84, 86, 88) angeordnet sind, von denen wenigstens zwei (80, 82, 84, 86) am Armbereich (90) so zum Umgreifen des Unterarms angeordnet sind, dass der Unterarm wenigstens abschnittsweise sowohl ellenseitig als auch speichenseitig von den flügelartigen Abschnitten (80, 82, 84, 86) umgriffen ist, **dadurch gekennzeichnet, dass** wenigstens ein flügelartiger Abschnitt (88) am Handbereich (92) die Hand ellenseitig zumindest teilweise umgreift.

2. Handgelenk-Orthese nach Anspruch 1, **dadurch gekennzeichnet, dass** die Schiene (12) vier flügelartige Abschnitte (80, 82, 84, 86) am Armbereich (90) und einen flügelartigen Abschnitt (88) am Handbereich (92) aufweist.

3. Handgelenk-Orthese nach Anspruch 1 oder 2, **dadurch gekennzeichnet, dass** die Schiene (12) einen länglich ausgebildeten Mittelsteg (78) aufweist, der sich über den Armbereich (90) und den Handbereich (92) der Schiene (12) erstreckt und mit dem die flügelartigen Abschnitte (80, 82, 84, 86, 88), insbesondere an den Längsseiten (94, 96) des Mittelsteges (78) einstückig, verbunden sind.

4. Handgelenk-Orthese nach einem der vorstehenden Ansprüche, **dadurch gekennzeichnet, dass** die Schiene (12) aus einem Metall, insbesondere aus Aluminium, und/oder aus einem Kunststoff besteht.

5. Handgelenk-Orthese nach einem der vorstehenden Ansprüche, **dadurch gekennzeichnet, dass** sich der flügelartige Abschnitt (88) am Handbereich (92) ausgehend vom Bereich der Handinnenfläche über den Bereich der Handkante (218) und zumindest teilweise bis in den Bereich des Handrückens (214) erstreckt.

6. Handgelenk-Orthese nach einem der vorstehenden Ansprüche, **dadurch gekennzeichnet, dass** die flügelartigen Abschnitte (80, 82, 84, 86, 88) zum Anpassen an die Kontur des Arms beziehungsweise die Kontur der Hand biegbar sind.

7. Handgelenk-Orthese nach einem der vorstehenden Ansprüche, **dadurch gekennzeichnet, dass** die flügelartigen Abschnitte (80, 82, 84, 86, 88) rahmenartig ausgebildet sind und eine von dem Rahmen (102, 104, 106, 108, 110) begrenzte Ausnehmung (112, 114, 116, 118, 120) aufweisen.

8. Handgelenk-Orthese nach einem der Ansprüche 3 bis 7, **dadurch gekennzeichnet, dass** der Mittelsteg (78) der Schiene (12) im Wesentlichen parallel zum Unterarm verläuft.

9. Handgelenk-Orthese nach einem der vorstehenden Ansprüche, **dadurch gekennzeichnet, dass** die Schiene (12) an den Verlauf der Kontur von Unterarm, Handgelenk und Mittelhand anpassbar und/oder angepasst ist, insbesondere in deren anatomischer Normalstellung.

10. Handgelenk-Orthese nach einem der vorstehenden Ansprüche, **dadurch gekennzeichnet, dass** der Handbereich (92) an die Form der Handinnenfläche angepasst ist, insbesondere tellerartig, O-förmig, L-förmig, T-förmig oder V-förmig.

11. Handgelenk-Orthese nach einem der vorstehenden Ansprüche, **dadurch gekennzeichnet, dass** die Schiene (12) wenigstens teilweise mit Leder und/oder einem Textil oder dergleichen überzogen ist, dass der Überzug (14) zumindest abschnittsweise atmungsaktiv und/oder feuchtigkeitsableitend ist und dass der Überzug (14) zumindest teilweise mit einer Polsterung versehen ist, insbesondere im Bereich der Schiene (12) und/oder den flügelartigen Abschnitten (80, 82, 84, 86, 88).

12. Handgelenk-Orthese nach einem der vorstehenden Ansprüche, **dadurch gekennzeichnet, dass** an der Handgelenk-Orthese (10) Verschlussmittel (18, 20, 22, 24, 26, 36, 38, 48, 50, 56, 66, 68, 74, 76) zum Fixieren der Orthese (10) vorgesehen sind, die wenigstens im Bereich des Unterarms und/oder im Bereich der Hand im Wesentlichen an der Seite (28) an der Handgelenk-Orthese angeordnet ist, die in der anatomischen Normalstellung der Rumpfmittelebene abgewandt ist, und dass mittels der Verschlussmittel (18, 20, 22, 24, 26, 36, 38, 48, 50, 56, 66, 68, 74, 76) eine Fixierung im wesentlichen über die gesamte Länge der Handgelenk-Orthese (10) erreicht werden kann.

13. Handgelenk-Orthese nach Anspruch 12, **dadurch gekennzeichnet, dass** die Verschlussmittel (18, 20, 22, 24, 26, 36, 38, 48, 50, 56, 66, 68, 74, 76) wenigstens ein Band (18, 20, 22, 24, 26), wenigstens einen Riemen und/oder einen Haken aufweisen, wobei das wenigstens eine Band (18, 20, 22, 24, 26), der wenigstens eine Riemen und/oder der Haken mit wenigstens einer Schlaufe (36, 38) in Eingriff steht, die an den flügelartigen Abschnitten (80, 82, 84, 86, 88), dem Überzug (14) und/oder der Schiene (12) angeordnet ist und dass das wenigstens eine Band (18, 20, 22, 24, 26) und der wenigstens eine Riemen mit einem Klettband (48, 50, 56, 66, 68) beziehungsweise einer Schnalle versehen ist.

14. Handgelenk-Orthese nach Anspruch 12 oder 13, **dadurch gekennzeichnet, dass** die Handgelenk-Orthese (10) mehrere Bänder (18, 20, 22, 24, 26) und/oder Riemen aufweist, die beabstandet zueinander im Wesentlichen senkrecht zum Unterarm im Armbereich (90) der Schiene (12) angeordnet sind, wobei wenigstens ein erstes Band (18, 20) oder Riemen an der der Elle zugewandten Seite (46, 96) der Schiene (12) fixiert ist und wenigstens ein zweites Band (22) oder Riemen an der der Speiche zugewandten Seite (40, 94) der Schiene (12) fixiert ist.

15. Handgelenk-Orthese nach Anspruch 14, **dadurch gekennzeichnet, dass** im Armbereich (92) wenigstens eines der Bänder (18, 20, 22, 24, 26) im verschlossenen Zustand den Arm im Bereich des Handgelenks vollständig umgibt und so breit ist, dass es den Bereich des Handgelenks in Längsrichtung des Arms gesehen im wesentlichen vollständig überdeckt.

16. Handgelenk-Orthese nach einem der vorstehenden Ansprüche, **dadurch gekennzeichnet, dass** eine Daumen-Schlaufe (24, 26, 60, 70) für die Fixierung der Handgelenk-Orthese (10) relativ zum Daumen vorgesehen ist.

17. Handgelenk-Orthese nach Anspruch 16, **dadurch gekennzeichnet, dass** die Daumen-Schlaufe (24, 26, 60, 70) aus einem Streifen (60) des Materials eines Überzugs (14) gebildet ist, wobei ein erster Endbereich des Streifens (60) in einem rumpffernen Endbereich (58) der Handgelenk-Orthese (10) mit dem Überzug (14) unlösbar verbunden ist und der Streifen (18) in einem zweiten Endbereich (62) mit der Handgelenk-Orthese (10) lösbar verbunden ist, insbesondere mittels eines Klettbandes (66, 68) oder dergleichen.

18. Handgelenk-Orthese nach Anspruch 17, **dadurch gekennzeichnet, dass** an dem zweiten Endbereich (62) des Streifens (60) zwei Bänder (66, 68), insbesondere Klettbänder, angeordnet sind, wobei eines der Bänder (66) den Streifen (60) im Wesentlichen verlängernd angeordnet ist und das zweite Band (68) in einen Winkel α zum ersten Band (66) ausgerichtet am Streifen (60) angeordnet ist.

19. Handgelenk-Orthese nach Anspruch 18, **dadurch gekennzeichnet, dass** das erste Band (66) und das zweite Band (68) einen Winkel α zwischen 100° und 160° einschließen, insbesondere 125°.

20. Handgelenk-Orthese nach Anspruch 18 oder 19, **dadurch gekennzeichnet, dass** das erste Band (24) im verschlossenen Zustand im Wesentlichen parallel zu einer Linie (142) ausgerichtet ist, die sich von einem Bereich (204) zwischen den Metakarpal-Phalangelenken (206, 208) von Daumen (210) und Zeigefinger (212) über den Handrücken (214) im Bereich der Mittelhandknochen (216) bis zur Handkante (218) im Bereich des Mittelhandknochens (220) des kleinen Fingers (222) erstreckt.

21. Handgelenk-Orthese nach wenigstens einem der Ansprüche 18 bis 20, **dadurch gekennzeichnet, dass** das zweite Band (26) im verschlossenen Zustand im Wesentlichen parallel zu einer Linie (144) ausgerichtet ist, die vom Metakarpal-Phalangelenk (224) des kleinen Fingers (222) über den Handrücken (214) zum Karpo-Metakarpalgelenk (226) des Daumens (210) verläuft.

22. Handgelenk-Orthese nach wenigstens einem der Ansprüche 18 bis 21, **dadurch gekennzeichnet, dass** die beiden Bänder (24, 26, 66. 68) als Klettbänder ausgebildet sind und an einem textilen Überzug (14) der Schiene (12) befestigbar sind.
